# FASCICULE DE BREVET EUROPEEN

(11) **EP 3 829 448 B1**
(45) Date de publication et mention de la délivrance du brevet: **07.06.2023**
(21) Numéro de dépôt: 19745604.9
(22) Date de dépôt: 26.07.2019
(51) Int. Cl.: A61B 10/00, A61G 9/00, A61M 1/00, A61B 17/22

(54) **DISPOSITIF PERMETTANT DE COLLECTER D'ÉVENTUELS CALCULS RÉNAUX PRÉSENTS DANS UN FLUX D'URINE, DESTINÉ ESSENTIELLEMENT À UN UTILISATEUR MASCULIN**
VORRICHTUNG ZUM SAMMELN MÖGLICHER NIERENSTEINE IM HARNSTRAHL, IM WESENTLICHEN FÜR EINEN MÄNNLICHEN BENUTZER
DEVICE FOR COLLECTING POSSIBLE KIDNEY STONES PRESENT IN A URINE STREAM, ESSENTIALLY INTENDED FOR A MALE USER

(30) Priorité: 01.08.2018 FR 1827229
(43) Date de publication de la demande: 09.06.2021
(73) Titulaire: Innospark, 38200 Vienne (FR)
(72) Inventeur: Vinck, Frédéric, 38200 Vienne (FR); Llerena, Joël, 13340 Rognac (FR)
(74) Mandataire: Opilex
(86) Numéro de dépôt international: PCT/EP2019/070180
(87) Numéro de publication internationale: WO 2020/025476

(56) Documents cités:
- FR-A1- 2 786 406
- US-A- 4 064 760
- US-A- 5 762 071
- US-B1- 6 235 010

## Description

La présente invention concerne un dispositif permettant de collecter d'éventuels calculs rénaux présents dans un flux d'urine, destiné essentiellement à un utilisateur masculin. Ce dispositif est destiné essentiellement à un utilisateur masculin, mais pas exclusivement : il serait en effet concevable qu'il soit utilisable par une femme urinant debout, quand les circonstances ne lui permettent pas de faire autrement, moyennant l'utilisation d'un accessoire tel que connu sous les dénominations "pisse-debout" ou "urinelle".

Il est utile de collecter d'éventuels calculs rénaux présents dans un flux d'urine. Les moyens employés à cet effet sont souvent très empiriques (filtre à café, filtre à thé, passoire, gaze,...) et présentent de nombreux inconvénients d'utilisation.

Il existe sinon des dispositifs de collecte dédiés, présentant un corps dans le fond duquel s'étend un tamis ; chaque ouverture que comprend ce tamis a une taille suffisante pour permettre le passage de l'urine au travers d'elle mais insuffisante pour permettre le passage d'éventuels calculs rénaux. Ces dispositifs dédiés ne donnent cependant pas satisfaction, ayant pour inconvénient de ne pas parfaitement éliminer les risques que de l'urine se répande sur l'extérieur du dispositif et viennent salir un environnement de la collecte (WC, parties du corps exposées, vêtements) ou un environnement du transport (poche, sac dans lequel le dispositif est replacé après utilisation).

La présente invention a pour objectif de fournir un dispositif remédiant à cet inconvénient essentiel, tout en conservant une structure simple et donc peu onéreuse à fabriquer, et une utilisation facile.

Les publications de brevet No. FR 2 786 406 A1, US 6,235,010 B1 et US 5,762,071 A illustrent différents dispositifs conforme à l'art antérieur. Le document No. FR 2 786 406 A1 décrit un dispositif comprenant un corps de récipient tubulaire, un tamis placé à l'intérieur de ce corps et deux couvercles d'extrémité permettant de sélectivement clore ou libérer les ouvertures d'extrémité du corps de récipient, le tamis s'étendant en retrait de l'extrémité inférieure du corps de récipient.

Le dispositif concerné comprend, de manière connue en soi, un corps de récipient tubulaire, un tamis placé à l'intérieur de ce corps et deux couvercles d'extrémité permettant de sélectivement clore ou libérer les ouvertures d'extrémité du corps de récipient, le tamis s'étendant en retrait de l'extrémité inférieure du corps de récipient.

Selon l'invention, le dispositif comprend, immédiatement en dessous du tamis, un entonnoir de guidage du flux d'urine dont l'ouverture distante du tamis est située à distance des parois du corps de récipient.

Il se comprend que le terme "inférieure" est à considérer selon la direction d'écoulement du flux d'urine au travers du dispositif, ce terme désignant une partie située, en position d'utilisation, en dessous d'une partie "supérieure" selon cette direction d'écoulement.

L'entonnoir selon l'invention permet ainsi de reconstituer le jet d'urine et de le maintenir à distance des parois du corps de récipient ; il permet par conséquent d'éliminer le risque que le flux d'urine se répande sur les parois du corps de récipient et donc de salir ce corps de récipient et l'environnement de collecte ou de transport du dispositif après cette collecte.

En cas de réussite de la collecte, il suffit de refermer le dispositif pour la conservation, le transport et la remise au laboratoire.

Lors des usages qui auront précédé la collecte, car elle n'est que rarement possible dès la première fois, il est possible de rincer ou nettoyer le dispositif.

Aucune des parties du dispositif ne peut se détacher des autres, que le dispositif soit ouvert ou fermé.

Le dispositif selon l'invention présente également les avantages suivants :
- il permet le séchage des calculs ;
- il évite les écoulements résiduels ;
- il évite de reprendre les calculs à la main (en comparaison avec les dispositifs sommaires existants sur le marché) ;
- il est intéressant pour les laboratoires d'analyses qui veulent être accrédités par l'organisme Cofrac ;
- il contribue à lutter contre les infections nosocomiales et la contamination de l'entourage du patient.

De préférence, l'entonnoir présente une hauteur telle que son ouverture distante du tamis soit située au niveau de la partie inférieure du corps de récipient ou proche de cette partie inférieure.

Le flux d'urine est, de cette façon, maintenu à distance de la paroi du corps de récipient jusqu'au niveau de la partie inférieure du corps de récipient, renforçant la prévention de tout risque que de l'urine vienne salir le corps de récipient, même lorsque le dispositif est maintenu dans une position inclinée d'utilisation.

L'entonnoir pourrait être purement conique ; selon une forme de réalisation préférée de l'invention, toutefois, l'entonnoir présente, du côté du tamis, une partie de forme conique et, du côté opposé au tamis, une partie de forme cylindrique ou conique raccordée à l'extrémité inférieure de la partie de forme conique.

Le tamis pourrait être conçu de façon à présenter une forme conique au niveau de son côté supérieur.

Le tamis et/ou l'entonnoir pourraient être constitués par des pièces réalisées de façon séparée du corps de récipient et être fixées à l'intérieur de ce corps après réalisation ; de préférence, toutefois, ce tamis et/ou cet entonnoir sont réalisés en une seule pièce avec le corps de récipient et sont donc d'un seul tenant avec celui-ci.

Le corps de récipient, le tamis et/ou l'entonnoir et/ou les couvercles peuvent ainsi être obtenus par une seule opération de moulage, donc de façon rapide et relativement peu onéreuse.

Au moins un des couvercles d'extrémité pourrait être monté sur le corps de récipient par vissage ou par tout autre moyen de montage amovible approprié ; de préférence, toutefois, chacun des couvercles d'extrémité est dimensionné de façon à être apte à mis en place sur le corps de récipient par emmanchement et à être maintenu sur ce corps par emboitement, de façon que la manipulation du dispositif soit simple. Selon une forme de réalisation préférée de l'invention dans ce cas, chaque couvercle est relié à une bague de maintien par une charnière-film permettant le pivotement du couvercle entre une position de fermeture, dans laquelle le couvercle est rapproché de la bague de maintien, et une position d'ouverture, dans laquelle le couvercle est éloigné de la bague de maintien, l'ensemble formé par le couvercle, la charnière-film et la bague de maintien étant réalisé en une pièce en matière synthétique déformable élastiquement ; le corps de récipient forme sur sa face externe, à proximité de chacune de ses extrémités longitudinales, une surépaisseur faisant saillie de sa face externe ; cette surépaisseur forme une gorge aménagée à l'intérieur d'elle et, entre cette gorge et l'extrémité longitudinale correspondante du corps, forme également une paroi inclinée constituant une rampe d'étirement élastique circonférentiel de la bague de maintien ; la bague de maintien est ainsi destinée à être engagée sur ladite surépaisseur, à subir un étirement circonférentiel élastique lors de son passage sur ladite paroi inclinée puis à être engagée dans ladite gorge ; la bague, une fois dans la gorge, retrouve sa circonférence de non-étirement par rappel élastique de son matériau constitutif et est donc retenue dans la gorge par encliquetage dans cette gorge.

Le montage de chaque couvercle est ainsi simple et rapide à réaliser.

Pour une étanchéité parfaite entre chaque couvercle et le corps de récipient, de préférence, chaque couvercle forme une jupe intérieure destinée à venir s'emmancher dans l'extrémité correspondante du corps de récipient.

Pour une parfaite rétention de chaque couvercle sur l'extrémité correspondante du corps de récipient, de préférence, le corps de récipient forme, au niveau de cette extrémité, un bourrelet externe arrondi et le couvercle présente une paroi de ceinture destinée à venir s'engager avec frottements sur ce bourrelet.

Avantageusement, chaque couvercle présente une patte de manipulation faisant saillie radialement de lui-même, vers l'extérieur, située sur le côté du couvercle opposé à la charnière-film.

Cette patte permet une ouverture facile du couvercle.

L'invention sera bien comprise, et d'autres caractéristiques et avantages de celle-ci apparaîtront, en référence au dessin schématique annexé, représentant, à titre d'exemple non limitatif, une forme de réalisation préférée du dispositif concerné.
La figure 1 est une vue de ce dispositif en perspective, dans un état de fermeture de deux couvercles qu'il comprend ;
la figure 2 en est une vue en perspective dans un état d'ouverture de ces mêmes couvercles ;
la figure 3 en est une vue similaire à la figure 2, en coupe longitudinale passant par l'axe d'un corps de récipient tubulaire qu'il comprend ; et
la figure 4 est une vue du corps de récipient seul, de côté, en coupe longitudinale passant par l'axe de ce corps.

Les figures représentent un dispositif 1 permettant de collecter d'éventuels calculs rénaux présents dans un flux d'urine, ce dispositif 1 étant destiné à un utilisateur masculin.

Le dispositif 1 comprend un corps de récipient 2 tubulaire et deux ensembles 3 de fermeture des extrémités de ce corps 2.

Le corps de récipient 2 a un diamètre tel qu'il permet l'introduction d'un pénis en lui et peut avoir une longueur allant d'environ 40 mm à environ 150 mm. Il comprend un tamis 5 et un entonnoir 6.

Le tamis 5 s'étend transversalement à l'intérieur du corps de récipient 2, en retrait de l'extrémité inférieure de ce corps. Chacune des ouvertures qui forment ce tamis a une taille de l'ordre de 0,8 à 5 mm², qui est donc suffisamment grande pour permettre le passage du flux d'urine au travers d'elle mais qui est suffisamment petite pour retenir les éventuels calculs rénaux exploitables présents dans l'urine de l'utilisateur.

L'entonnoir 6 est situé immédiatement en dessous du tamis 5. Il présente une hauteur telle que son ouverture distante du tamis 5 soit située au niveau de la partie inférieure du corps 2. Il présente, du côté du tamis 5, une partie de forme conique et, du côté opposé à ce tamis, une partie de forme cylindrique raccordée à l'extrémité inférieure de la partie de forme conique. L'ouverture de l'entonnoir 6 distante du tamis 5 est ainsi située radialement à distance des parois du corps de récipient 2.

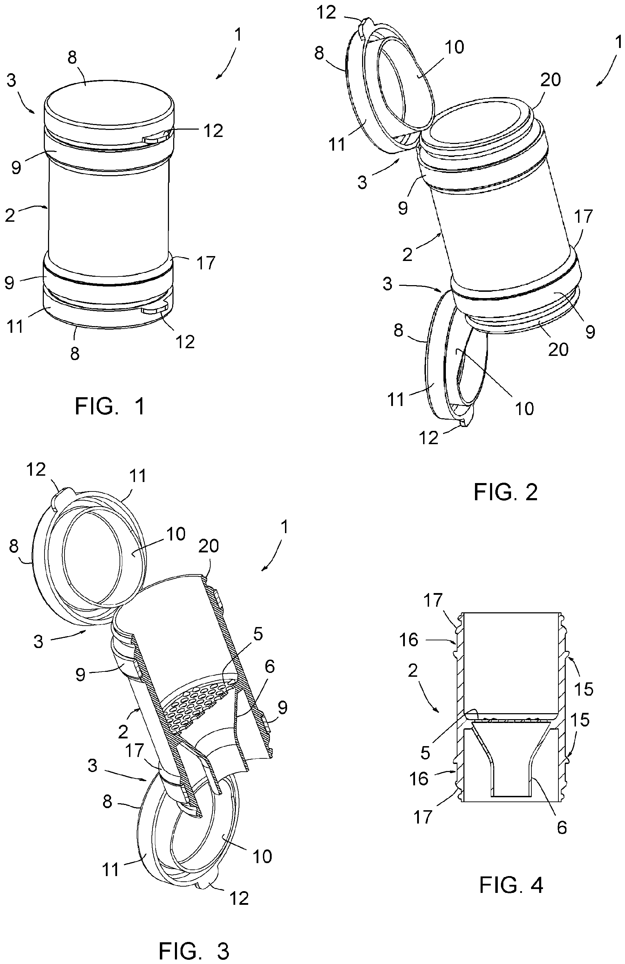

Comme visible sur les figures 3 et 4, le tamis 5 et l'entonnoir 6 sont réalisés en une seule pièce avec le corps de récipient 2 et sont donc d'un seul tenant avec celui-ci.

Chaque ensemble de fermeture 3 comprend un couvercle 8 permettant de sélectivement clore ou libérer les ouvertures d'extrémité du corps de récipient 2, une bague de maintien 9 et une charnière-film (non visible sur les figures) reliant le couvercle 8 à la bague 9. L'ensemble 3 est réalisé en une pièce en matière synthétique légèrement déformable élastiquement, qui peut être toute matière synthétique courante appropriée.

Chaque couvercle 8 forme une jupe intérieure 10 destinée à venir s'emmancher dans l'extrémité correspondante du corps de récipient 2, une paroi de ceinture 11 et une patte de manipulation 12 faisant saillie radialement de lui-même, vers l'extérieur, cette patte 12 étant située sur le côté du couvercle 8 opposé à la charnière-film.

La bague 9 est simplement formée par un anneau ; le diamètre interne de cet anneau correspondant au diamètre que présente le corps de récipient 2 au niveau d'une gorge 16 décrite ci-après. La bague 9, du fait de la légère déformabilité élastique de son matériau constitutif, peut-être légèrement déformée élastiquement selon sa circonférence.

Comme visible particulièrement sur la figure 4, le corps de récipient 2 forme sur sa face externe, à proximité de chacune de ses extrémités longitudinales, une surépaisseur 15 faisant saillie de sa face externe ; cette surépaisseur 15 forme ladite gorge 16, aménagée à l'intérieur d'elle, et, entre cette gorge 16 et l'extrémité longitudinale correspondante du corps 2, une paroi inclinée 17 constituant une rampe d'étirement élastique circonférentiel de la bague de maintien 9.

La bague de maintien 9 est ainsi destinée à être engagée sur la surépaisseur 15, à subir un étirement circonférentiel élastique lors de son passage sur la paroi inclinée 17 puis à être engagée dans la gorge 16 ; la bague 9, une fois dans la gorge 16, retrouve sa circonférence de non-étirement par rappel élastique de son matériau constitutif et est donc retenue dans la gorge 16 par encliquetage dans cette gorge.

Comme cela se comprend en référence aux figures 1 et 2, chaque bague 9 permet le pivotement du couvercle 8 correspondant entre la position de fermeture montrée sur la figure 1, dans laquelle le couvercle 8 est rapproché de la bague de maintien 9 et ferme l'ouverture correspondante du corps 2, et la position d'ouverture montrée sur la figure 2, dans laquelle le couvercle 8 est éloigné de la bague de maintien 9 et libère ladite ouverture pour permettre l'utilisation du dispositif 1, à savoir pour permettre l'introduction du pénis de l'utilisateur par l'ouverture supérieure du corps de récipient 2 et l'évacuation de l'urine par l'ouverture inférieure de ce corps.

En position de fermeture, la jupe intérieure 10 de chaque couvercle 8 vient s'emmancher dans l'extrémité correspondante du corps de récipient 2, permettant ainsi une étanchéité parfaite entre le couvercle 8 et le corps 2, et la paroi de ceinture 11 que forme le couvercle 8 vient s'engager avec frottements sur un bourrelet 20 que forme l'extrémité correspondante du corps de récipient 2, pour une parfaite rétention du couvercle 8 en position de fermeture. La patte 12 permet quant à elle une ouverture facile du couvercle 8.

Les couvercles se maintiennent ouverts grâce à la forme particulière de la jupe 10, en appui sur le col du corps.

Comme cela se comprend, l'entonnoir 6 permet de reconstituer le jet d'urine et de le maintenir à distance de la paroi du corps de récipient 2, jusqu'au niveau de la partie inférieure de cette paroi. Grâce au fait que l'on peut ainsi facilement diriger le jet, par exemple pour viser l'intérieur de la cuvette des toilettes, tout risque que l'urine vienne salir un environnement de la collecte (WC, parties du corps exposées, vêtements) est ainsi éliminé.

Le montage de chaque couvercle 8 est par ailleurs simple et rapide à réaliser, et le dispositif est relativement simple et rapide à assembler. En outre, l'utilisation du dispositif est simple et intuitive.

Le dispositif 1 selon l'invention permet de cette façon de remédier à plusieurs inconvénients notables des dispositifs homologues existants.

L'invention a été décrite ci-dessus en référence à une forme de réalisation fournie uniquement à titre d'exemple. Il va de soi qu'elle n'est pas limitée à cette forme de réalisation et qu'elle s'étend à toutes les autres formes de réalisation couvertes par les revendications ci-annexées. Ainsi, le dispositif 1 pourrait comporter latéralement une poignée ou un manche lui permettant d'être tenu par une tierce personne si nécessaire ; le tamis 5 pourrait présenter une structure en nid d'abeille, et pourrait être bombé ; le dispositif pourrait inclure un PH-mètre et/ou un moyen de traçabilité par exemple sous la forme d'un étiquetage ou d'un système RFID.

## Revendications

1. Dispositif (1) permettant de collecter d'éventuels calculs rénaux présents dans un flux d'urine, destiné à un utilisateur masculin, ce dispositif (1) comprenant un corps de récipient (2) tubulaire, un tamis (5) placé à l'intérieur de ce corps et deux couvercles d'extrémité (8) permettant de sélectivement clore ou libérer les ouvertures d'extrémité du corps de récipient (2), le tamis (5) s'étendant en retrait de l'extrémité inférieure du corps de récipient (2).
**caractérisé en ce que** le dispositif (1) comprend, immédiatement en dessous du tamis (5), un entonnoir (6) de guidage du flux d'urine dont l'ouverture distante du tamis (5) est située à distance des parois du corps de récipient (2).

2. Dispositif (1) selon la revendication 1, **caractérisé en ce que** l'entonnoir (6) présente une hauteur telle que son ouverture distante du tamis (5) soit située au niveau de la partie inférieure du corps de récipient (2) ou proche de cette partie inférieure.

3. Dispositif (1) selon la revendication 1 ou la revendication 2, **caractérisé en ce que** l'entonnoir (6) présente, du côté du tamis (5), une partie de forme conique et, du côté opposé au tamis (5), une partie de forme cylindrique ou conique raccordée à l'extrémité inférieure de la partie de forme conique.

4. Dispositif (1) selon l'une des revendications 1 à 3, **caractérisé en ce que** le tamis (5) est conçu de façon à présenter une forme conique au niveau de son côté supérieur.

5. Dispositif (1) selon l'une des revendications 1 à 4, **caractérisé en ce que** le tamis (5) et/ou l'entonnoir (6) sont réalisés en une seule pièce avec le corps de récipient (2) et sont donc d'un seul tenant avec celui-ci.

6. Dispositif (1) selon l'une des revendications 1 à 5, **caractérisé en ce que** chacun des couvercles d'extrémité (8) est dimensionné de façon à être apte à être mis en place sur le corps de récipient (2) par emmanchement et à être maintenu sur ce corps par emboitement.

7. Dispositif (1) selon la revendication 6, **caractérisé en ce que** chaque couvercle (8) est relié à une bague de maintien (9) par une charnière-film permettant le pivotement du couvercle (8) entre une position de fermeture, dans laquelle le couvercle (8) est rapproché de la bague de maintien (9), et une position d'ouverture, dans laquelle le couvercle (8) est éloigné de la bague de maintien (9), l'ensemble (3) formé par le couvercle (8), la charnière-film et la bague de maintien (9) étant réalisé en une pièce en matière synthétique déformable élastiquement ; le corps de récipient (2) forme sur sa face externe, à proximité de chacune de ses extrémités longitudinales, une surépaisseur (15) faisant saillie de sa face externe ; cette surépaisseur forme une gorge (16) aménagée à l'intérieur d'elle et forme également, entre cette gorge et l'extrémité longitudinale correspondante du corps de récipient (2), une paroi inclinée (17) constituant une rampe d'étirement élastique circonférentiel de la bague de maintien (9) ; la bague de maintien (9) est ainsi destinée à être engagée sur ladite surépaisseur (15), à subir un étirement circonférentiel élastique lors de son passage sur ladite paroi inclinée (17) puis à être engagée dans ladite gorge (16) ; la bague (9), une fois dans la gorge (16), retrouve sa circonférence de non-étirement par rappel élastique de son matériau constitutif et est donc retenue dans la gorge (16) par encliquetage dans cette gorge.

8. Dispositif (1) selon la revendication 6 ou la revendication 7, **caractérisé en ce que** chaque couvercle (8) forme une jupe intérieure (10) destinée à venir s'emmancher dans l'extrémité correspondante du corps de récipient (2).

9. Dispositif (1) selon l'une des revendications 6 à 8, **caractérisé en ce que** le corps de récipient (2) forme, au niveau de cette extrémité, un bourrelet externe arrondi (20) et **en ce que** le couvercle (8) présente une paroi de ceinture (11) destinée à venir s'engager avec frottements sur ce bourrelet.

10. Dispositif (1) selon l'une des revendications 7 à 9, **caractérisé en ce que** chaque couvercle (8) présente une patte de manipulation (12) faisant saillie radialement de lui-même, vers l'extérieur, cette patte de manipulation (12) étant située sur le côté du couvercle (8) opposé à la charnière-film.

## Patentansprüche

1. Vorrichtung (1) zum Sammeln möglicher Nierensteine, die in einem Urinstrom vorhanden sind, die für einen männlichen Benutzer bestimmt ist, die Vorrichtung (1) umfassend einen rohrförmigen Behälterkörper (2), ein im Inneren dieses Körpers platziertes Sieb (5) und zwei Enddeckel (8), die ein wahlweises Schließen oder Freigeben der Endöffnungen des Behälterkörpers (2) ermöglichen, wobei sich das Sieb (5) von dem unteren Ende des Behälterkörpers (2) zurückversetzt erstreckt,
**dadurch gekennzeichnet, dass** die Vorrichtung (1) unmittelbar unterhalb des Siebs (5) einen Trichter (6) zum Leiten des Urinstroms umfasst, dessen von dem Sieb (5) beabstandete Öffnung sich in einem Abstand von den Wänden des Behälterkörpers (2) befindet.

2. Vorrichtung (1) nach Anspruch 1, **dadurch gekennzeichnet, dass** der Trichter (6) eine solche Höhe aufweist, dass sich seine von dem Sieb (5) beabstandete Öffnung an oder nahe dem unteren Abschnitt des Behälterkörpers (2) befindet.

3. Vorrichtung (1) nach Anspruch 1 oder Anspruch 2, **dadurch gekennzeichnet, dass** der Trichter (6) auf der Seite des Siebs (5) einen konisch geformten Abschnitt und auf der dem Sieb (5) gegenüberliegenden Seite einen zylindrisch oder konisch geformten Abschnitt aufweist, der mit dem unteren Ende des konisch geformten Abschnitts verbunden ist.

4. Vorrichtung (1) nach einem der Ansprüche 1 bis 3, **dadurch gekennzeichnet, dass** das Sieb (5) derart gestaltet ist, dass es an seiner Oberseite eine konische Form aufweist.

5. Vorrichtung (1) nach einem der Ansprüche 1 bis 4, **dadurch gekennzeichnet, dass** das Sieb (5) und/oder der Trichter (6) einstückig mit dem Behälterkörper (2) ausgebildet sind und somit einteilig damit sind.

6. Vorrichtung (1) nach einem der Ansprüche 1 bis 5, **dadurch gekennzeichnet, dass** jeder der Enddeckel (8) derart bemessen ist, dass er geeignet ist, auf dem Behälterkörper (2) platziert zu werden, indem er eingesteckt wird und durch Verfalzen an diesem Körper gehalten wird.

7. Vorrichtung (1) nach Anspruch 6, **dadurch gekennzeichnet, dass** jeder Deckel (8) mit einem Haltering (9) über ein Filmscharnier verbunden ist, das das Schwenken des Deckels (8) zwischen einer Schließposition, in der der Deckel (8) an den Haltering (9) angenähert ist, und einer Öffnungsstellung, in der der Deckel (8) vom Haltering (9) entfernt ist, ermöglicht, wobei die aus dem Deckel (8), dem Filmscharnier und dem Haltering (9) gebildete Anordnung (3) aus einem elastisch verformbaren Kunststoffteil hergestellt ist; wobei der Behälterkörper (2) auf seiner Außenseite in der Nähe von jedem seiner Längsenden eine Verdickung (15) bildet, die von seiner Außenseite hervorsteht; diese Verdickung eine im Inneren davon angeordnete Nut (16) bildet und zwischen dieser Nut und dem entsprechenden Längsende des Behälterkörpers (2) auch eine geneigte Wand (17) bildet, die eine Rampe für eine elastische Umfangsdehnung des Halterings (9) bildet; wobei der Haltering (9) somit dazu bestimmt ist, auf der genannten Verdickung (15) in Eingriff gebracht zu werden, bei seinem Durchgang durch die genannte geneigte Wand (17) eine elastische Umfangsdehnung zu erfahren und dann in der genannten Nut (16) in Eingriff gebracht zu werden; wobei der Ring (9), sobald er in der Nut (16) ist, durch elastische Rückstellung seines konstituierenden Materials zu seinem Nicht-Dehnungsumfang zurückkehrt und somit durch Einrasten in dieser Nut in der Nut (16) gehalten wird.

8. Vorrichtung (1) nach Anspruch 6 oder Anspruch 7, **dadurch gekennzeichnet, dass** jeder Deckel (8) eine innere Schürze (10) bildet, die dazu bestimmt ist, in das entsprechende Ende des Behälterkörpers (2) gesteckt zu werden.

9. Vorrichtung (1) nach einem der Ansprüche 6 bis 8, **dadurch gekennzeichnet, dass** der Behälterkörper (2) auf Ebene dieses Endes einen abgerundeten äußeren Wulst (20) bildet und dass der Deckel (8) eine Gürtelwand (11) aufweist, die dazu bestimmt ist, reibschlüssig an diesem Wulst einzugreifen.

10. Vorrichtung (1) nach einem der Ansprüche 7 bis 9, **dadurch gekennzeichnet, dass** jeder Deckel (8) eine Manipulationslasche (12) aufweist, die radial davon nach außen hervorsteht, wobei sich diese Manipulationslasche (12) auf der Seite des Deckels (8) gegenüber dem Filmscharnier befindet.

## Claims

1. Device (1) allowing the collection of possible kidney stones present in a urine stream, intended for a male user, said device (1) comprising a tubular container body (2), a screen (5) placed inside this body and two end covers (8) allowing the end openings of the container body (2) to be selectively closed or opened, said screen (5) extending away from the lower end of the container body (2),
**characterized in that** the device (1) comprises, immediately below the screen (5), a funnel (6) for guiding the flow of urine, the opening of said funnel remote from the screen (5) being situated away from the walls of the container body (2).

2. Device (1) according to Claim 1, **characterized in that** the funnel (6) is of such a height that its opening remote from the screen (5) is situated at the lower portion of the container body (2) or close to said lower portion.

3. Device (1) according to Claim 1 or Claim 2, **characterized in that** the funnel (6) has a conical portion on the side of the screen (5) and a cylindrical or conical portion connected to the lower end of said conical portion on the side opposite the screen (5).

4. Device (1) according to one of Claims 1 to 3, **characterized in that** the screen (5) is designed so as to have a conical shape at its upper side.

5. Device (1) according to one of Claims 1 to 4, **characterized in that** the screen (5) and/or the funnel (6) are realized in a single piece with the container body (2) and are therefore in one piece therewith.

6. Device (1) according to one of Claims 1 to 5, **characterized in that** each of the end covers (8) is of such dimensions that it is capable of being placed on the container body (2) by press-fitting and being held on said body by interlocking.

7. Device (1) according to Claim 6, **characterized in that** each cover (8) is connected to a retaining ring (9) by a film hinge, allowing the cover (8) to pivot between a closed position, in which the cover (8) is brought close to the retaining ring (9), and an open position, in which the cover (8) is removed from the retaining ring (9), the assembly (3) formed by the cover (8), the film hinge and the retaining ring (9) being realized as a part made of elastically deformable synthetic material; the container body (2) forms on its outer face, close to each of its longitudinal ends, an allowance (15) projecting from its outer face; this allowance forms a groove (16) provided on the inside thereof and likewise forms, between this groove and the corresponding longitudinal end of the container body (2), a sloping wall (17) which constitutes a circumferential elastic stretching ramp of the retaining ring (9); the retaining ring (9) is thereby intended to be engaged on said allowance (15), to undergo elastic circumferential stretching when passing over said sloping wall (17) then to be engaged in said groove (16); the ring (9), once inside the groove (16), recovers its non-stretch circumference by elastic return of its constituent material and is therefore retained in the groove (16) by snap-fitting in said groove.

8. Device (1) according to Claim 6 or Claim 7, **characterized in that** each cover (8) forms an internal skirt (10) intended to be fitted into the corresponding end of the container body (2).

9. Device (1) according to one of Claims 6 to 8, **characterized in that** the container body (2) forms a rounded outer bead (20) at this end and the cover (8) exhibits an enclosing wall (11) intended to engage with friction on this bead.

10. Device (1) according to one of Claims 7 to 9, **characterized in that** each cover (8) has a handling tab (12) projecting radially from itself towards the outside, said handling tab (12) being situated on the cover side (8) opposite the film hinge.
